(19) **Europäisches Patentamt / European Patent Office / Office européen des brevets**

(11) **EP 1 597 623 B1**

(12) **EUROPEAN PATENT SPECIFICATION**

(45) Date of publication and mention
of the grant of the patent:
**28.08.2013 Bulletin 2013/35**

(51) Int Cl.:
*G02C 7/00* (2006.01)    *G02C 7/04* (2006.01)
*A61B 3/103* (2006.01)    *G02C 7/02* (2006.01)

(21) Application number: **04710440.1**

(22) Date of filing: **12.02.2004**

(86) International application number:
**PCT/GB2004/000537**

(87) International publication number:
**WO 2004/072709 (26.08.2004 Gazette 2004/35)**

(54) **METHODS FOR DESIGNING CUSTOM LENSES FOR IMPROVED VISION AND CORRESPONDING LENSES**

METHODE ZUM DESIGN INDIVIDUELLER LINSEN FÜR EIN VERBESSERTES SEHVERMÖGEN UND ENTSPRECHENDE LINSEN

PROCEDES DE CONCEPTION DE LENTILLES PERSONNALISEES OFFRANT UNE VISION AMELIOREE ET LENTILLES CORRESPONDANTES

(84) Designated Contracting States:
**AT BE BG CH CY CZ DE DK EE ES FI FR GB GR HU IE IT LI LU MC NL PT RO SE SI SK TR**

(30) Priority: **12.02.2003 GB 0303193**

(43) Date of publication of application:
**23.11.2005 Bulletin 2005/47**

(73) Proprietor: **Guillon, Michel**
**London SW3 4AH (GB)**

(72) Inventors:
• **GUILLON, Michel**
**London SW3 4AH (GB)**

• **GOBBE, Marine Emmanuelle**
**Kingston-Upon-Thames,**
**Surrey KT1 2LH (GB)**

(74) Representative: **Smaggasgale, Gillian Helen**
**WP Thompson**
**55 Drury Lane**
**London WC2B 5SQ (GB)**

(56) References cited:
WO-A-02/088830      WO-A-03/032825
WO-A-2004/023990    US-B1- 6 305 802
US-B1- 6 338 559    US-B1- 6 499 843

## Description

The present invention relates to a method for designing lenses, particularly contact lenses, to correct vision.

Correction of vision includes an improvement in vision when measured quantitatively by known techniques and/or to the qualitative improvement of "seeing better" as described by the subject.

A large proportion of the population has vision that is less than optimum due to the presence of refractive abnormalities (known as aberrations) in the eye. In the absence of aberrations, all rays of light from any point in object space that are refracted by the optical system of the eye, will focus at one point in the image plane. However, in the presence of aberrations, some of the rays do not focus at the expected image point but intersect the image plane in a spread-out pattern such that the quality of the image is degraded.

The most well known of these aberrations are defocus and astigmatism which are collectively referred to as refractive errors. These are known as second-order aberrations and are conventionally corrected by the use of spectacles, contact lenses, intra-ocular lenses, inlays, onlays and the like. Surgical procedures, which may be used to correct the second order aberrations, include cataract removal, keratoplasty (corneal replacement), laser assisted in-site keratomileusis (LASIK), laser epithelial keratomileusis (LASEK), photorefractive keratectomy (PRK) and the like. LASIK and LASEK involve sculpting the cornea using an excimer laser. Whilst these devices and surgical methods are able to assist and often correct these second-order aberrations, the eye may additionally include higher-order forms of aberrations which go beyond refractive error and which degrade the quality of the retinal image. It has been suggested that after defocus and astigmatism have been corrected it is these residual higher order aberrations that most affect visual performance.

These higher order aberrations include spherical aberrations and coma aberrations. Spherical aberrations occur where the lens does not focus parallel rays to a point but instead focuses them along a line and as such is described as the failure of rays of light to unite at the paraxial focus. The further a ray of light is from the optical axis, the further its axial crossing point is from the image plane. Coma aberrations occur because off-axis rays do not converge at the focal plane. Thus they are present at the fovea and are due to the lack of rotational symmetry of the eye about an appropriate reference axis. Other higher order aberrations may be present including secondary astigmatism, trefoil aberrations, tetrafoil aberrations and the like. These higher order aberrations may occur naturally or may be introduced during surgical techniques such as LASIK or LASEK or by pathological conditions such as keratoconus.

Many studies have been carried out to try to measure and analyse the monochromatic aberrations in the human eye. All authors are agreed that the aberrations differ greatly between patients and that they are dependant on the size of the pupil. With a view to improving treatments for patients and improving their vision, methods have been developed for correcting these higher order aberrations and it has been shown in the laboratory that contrast sensitivity and resolution can be improved. Recently customised refractive procedures have been used to correct individual aberrations. In general, these methods involve the measurement of higher order aberrations and the transfer of the data relating to the aberrations to either the machines which produce, for example, contact lenses or intra ocular lenses or to the laser software which is used in surgery to correct the eye. In this later example the transfer of data enables the corneal ablation profile to take into account not only the sphere and cylinder aberrations but also the higher-order aberrations such that the use of a small computer-controlled excimer spot laser enables local areas of the cornea to be ablated as required to correct the aberrations of the eye.

One example of the technique of measuring higher order aberrations and utilising the data to design lenses can be found in US 6499843. In the described processes, the aberrations present in a patient's eye are measured using ocular wavefront aberration measurement techniques. This data is then transmitted to custom contact lens manufacturing facility which produces lenses to the required specification. The measured wavefront aberrations are preferably third and higher order aberrations and more preferably up to fourth to tenth order aberrations.

Other examples of processes which measure these higher order aberrations include US 6086204, US 6338559 and US 6305802.

Whilst these methods offer appropriate methods for treating such higher order aberrations, they do not recognise that different types of higher order monochromatic aberrations produce different effects on visual performance.

The effects of defocus and astigmatism, which are reported in diopters, are well known to the clinician. However, unlike defocus, the effect of higher order aberrations, which are described in micrometers, on visual performance has not, to date, been known. The amount of higher order aberrations in the human eye is usually described by a single number known as the Root Mean Square (RMS) wavefront error. The RMS is calculated from individual Zernike coefficients. The aberrations of a general optical system can be represented by a wavefront aberration polynomial: $W(\rho,\theta)$, which value depends on the coordinates $(\rho,\theta)$ in the pupil plane. Zernike polynomials are used to describe aberrations, as they facilitate the description of higher order aberrations; they are a set of complete orthogonal polynomials defined on a unit circle. The Zernike polynomials can be conveniently written in polar coordinates $(\rho,\theta)$, where $\rho$ is the radial coordinate ranging from 0 to 1 and $\theta$ is the azimuthal component ranging from 0 to $2\Pi$. They are defined as:

$$Z_n^{\pm m} = \begin{cases} \sqrt{(2n+1)} \; R_n^m(\rho) \cos m\theta & \text{for } m > 0 \\ \sqrt{(2n+1)} \; R_n^m(\rho) \sin m\theta & \text{for } m < 0 \\ \sqrt{(n+1)} \; R_n^m(\rho) & \text{for } m = 0 \end{cases}$$

Each of the Zernike consists of three components: a normalization factor $\sqrt{(2n+1)}$, a radial dependent component ($R_n^m$), and an azimuthal component. The radial component is a polynomial function, whereas the azimuthal component is a sinusoidal function.

The wavefront can be written as follow:

$$W(\rho,\theta) = \sum Z_n * Z_n(\rho,\theta)$$

where Zn is the Zernike coefficient and Zn ($\rho,\theta$) the Zernike polynomial which depends on the pupil coordinates.

Whilst the RMS gives information about the amplitude of aberrations present, it does not give any information about the effect provided by the different components of the RMS. Thus when a clinician evaluates a RMS chart, the same importance is given to the RMS of each individual Zernike coefficient, regardless of the type of aberration present.

In conventional approaches the overall aberrations of the eye are measured, for example by videoaberroscopy, in some circumstances this is combined with a measurement of corneal front surface aberrations which may be measured by videokeratoscopy. From this measurement calculations can be made to correct all of the detected aberrations up to a certain order. Maximum orders are often identified as fourth, sixth and tenth. It is believed that the correction of these higher order aberrations provides a higher visual performance than can be achieved by conventional corrections.

However, one problem with this approach is that it does not consider whether the higher order aberrations present are high enough to produce a significant loss of visual performance such that their correction will not achieve a noticeable improvement in vision. A further problem is that it is assumed that optical effects and visual effects are synonymous such that the visual effect of the higher order aberrations is measured by the optical effect of these aberrations. It is now believed that these conventional viewpoints may be incorrect.

Another aspect overlooked by current technology is that the process of accurately correcting the higher order aberrations is complex involving firstly an accurate measurement of the aberrations and secondly an accurate correction. Both the measurement techniques and the correction techniques are not perfectly accurate or repeatable and thus it is now believed that it may not be appropriate to correct all aberrations measured regardless of their visual effects, due to the limited reliability of their determination. In particular it is noted that the measurement of individual corneal aberrations is not totally repeatable, the measurement of individual overall aberrations is not totally repeatable, that there is no standardised methodology to measure higher order aberrations of contact lenses as per the International Standard Organisation and that the resurfacing of the cornea by either surgical methods such as PRK or LASIK is not fully predictable. PRK produces an inflammatory corneal response that is variable amongst individual patients and leads to a final correction at times which is grossly different to the intended correction. LASIK produces the correction deep within the corneal tissues but its effect takes place at the corneal front surface and the molding of the surface where the treatment is applied by the overlying corneal flap is not fully understood.

The invention is defined by the appended claims.

It has now been discovered that the effects of higher order aberrations should be evaluated in terms of their effects on vision and their correction and the order of corrections should be decided based upon these criteria. In particular, it has now been discovered that the blur created by the same level of Zernike coefficient of each specific aberration will have a different optical effect and will not affect the visual performance in the same way. Similar conclusions can be drawn where alternative methods of considering higher order aberrations are used such as Point Spread Function are used. The present invention takes note of this and provides methods that enable a different importance to be attributed to each individual Zernike coefficient and in particular the visual effect of different higher order aberrations to be normalised in relation to the visual effect produced by defocus, the effects of which are well understood.

The visual effect of different higher order aberrations are normalised in relation to the visual effect produced by defocus.

In the methods of the present invention, a correcting factor is used that will normalise the RMS with regard to the effect on visual performance in order to obtain a Visual Performance Detrimental Factor (VPDF) rather than relying on the total RMS. To establish the relative visual effect of the different aberrations, vision test charts were distorted by different higher order aberrations, all distortion having an equal optical value defined by an equal RMS.

One method to normalise the visual effects of higher order aberrations is to deform images, for example test charts, with higher order aberrations of fixed optical effects, for example, the same level of wavefront error RMS. The effects can then be compared to the visual effects of such distortions with those produced with different levels of defocus. One alternative means of achieving the distorted images is to use a deformable mirror.

A test chart suitable for measuring the effects of higher order aberrations wherein the images are deformed with higher order aberrations of fixed optical effects, defined by equal Zernike or other optical means may be used in testing for the visual effects of single or groups of higher order aberrations

Test charts distorted by defocus of several RMS values as well as test charts distorted with higher order aberrations were produced. The relative readability of the charts was then measured by a test panel of subjects who read all the charts. The relative readability was quantified in terms of relative visual loss compared to an undistorted vision test chart viewed under the same conditions.

A model has been validated to establish the relative visual effect of the third and fourth orders aberrations as given below.

$$\text{RMS} = \text{SQRT}(\ 1.1*\ (Z_4^{-2})^{\ 2} + 1.1*(Z_4^{2})^{\ 2} + 0.7*\ (Z_3^{1})^{\ 2} + 0.7*\ (Z_3^{-1})^{\ 2} + 0.8*\ (Z_4^{0})^{\ 2}$$
$$+ 0.5*\ (Z_3^{-3})^{\ 2} + 0.5*\ (Z_3^{3})^{\ 2} + 0.3*(Z_4^{-4})^{\ 2} + 0.3*(Z_4^{4})^{\ 2}\ ).$$

A further model has been validated to establish the relative visual effect of the third, fourth, fifth and sixth order aberrations as given below.

$$\text{RMS} = \text{SQRT}(\ 1.1*\ (Z_4^{-2})^{\ 2} + 1.1*(Z_4^{2})^{\ 2} + 0.7*\ (Z_3^{1})^{\ 2} + 0.7*\ (Z_3^{-1})^{\ 2} + 0.8*\ (Z_4^{0})^{\ 2}$$
$$+ 0.5*\ (Z_3^{-3})^{\ 2} + 0.5*\ (Z_3^{3})^{\ 2} + 0.3*(Z_4^{-4})^{\ 2} + 0.3*(Z_4^{4})^{\ 2} + 1.2*\ (Z_6^{-2})^{\ 2} + 1.2*\ (Z_6^{+2})^{\ 2} +$$
$$1.1*\ (Z_5^{-3})^{\ 2} + 1.1*\ (Z_5^{+3})^{\ 2} + 1.0*\ (Z_5^{-1})^{\ 2} + 1.0*\ (Z_5^{+1})^{\ 2} + 0.9*\ (Z_6^{0})^{\ 2} + 0.9*\ (Z_6^{-4})^{\ 2}$$
$$+ 0.9*\ (Z_6^{+4})^{\ 2} + 0.5*\ (Z_5^{-5})^{\ 2} + 0.5*\ (Z_5^{5})^{\ 2} + 0.3*(Z_6^{-6})^{\ 2} + 0.3*(Z_6^{6})^{\ 2}\ ).$$

Models can be developed up to the tenth order. Different models may be arrived at by the same technical approach for different populations. These models fall within the scope of the present invention.

The VPDF is calculated for a given pupil as the loss in visual acuity compared to the best corrected visual performance. The following steps are used in order to calculate the VPDF:

- Calculation of visual loss compared to baseline performance, which is the best corrected visual acuity, for high contrast and low contrast letters.
  Visual Acuity loss = Best corrected Visual Acuity - Visual Performance measured
- Calculation of the mean Visual Acuity loss for high and low contrast charts Mean Visual Acuity loss = ((Visual Acuity loss High Contrast) + (Visual Acuity loss Low Contrast)) / 2
- Calculation of the mean visual acuity loss for all the individual Zernike coefficients
- Calculation of the VPDF for each individual Zernike coefficients

$$\text{VPDF}\ (Zx) = (\text{Mean Visual Acuity loss for } Zx) / (\text{Mean Visual Acuity loss for defocus})$$

VPDF can alternatively be developed for charts with different contrasts and alternative techniques to calculate visual loss can be used.

The VPDF is calculated for each individual Zernike coefficients and for specific pupil sizes.

By the recognition of the different effects provided by the various aberrations the clinician can decide which require treatment and which can be left untreated as their effect on vision is minimal. In particular, the decision as to whether or not to correct and which aberrations to correct include the quantification of the effect of correcting such higher order aberrations.

According to a first aspect of the present invention there is provided a method for designing a custom lens having a spherical back surface which is tailored for the relative visual effect of different types of aberrations. The method comprises the steps of:

(a) measuring total ocular higher order aberrations;
(b) calculating the front surface correction needed in terms of Zernike coefficients;
(c) converting the correction using the Visual Performance Detrimental Factor;
(d) obtaining the relevant higher order aberrations for correction; and
(e) obtaining the optimised design for the front surface of the lens.

The custom lens may be a contact lens, preferably a soft or rigid contact lens, an inlay, an onlay or an intra-ocular lens.

The total ocular higher order aberrations may be measured by any suitable method. Suitable methods include the use of a wavefront sensor but may include other techniques including phase diversity techniques. A particularly suitable method is described in US 6305802.

The front surface correction needed in terms of Zernike coefficients can also be calculated by any suitable technique and again a suitable technique is described in US 6305802.

The VPDF can then be used to obtain the relevant higher order aberrations for which correction will be appropriate and an appropriate lens can then be prepared by known techniques.

In a second embodiment of the present invention the VPDF is used to optimise the design of both the front and back surface of the lens. In this arrangement the method comprises the steps of:

(a) measuring total ocular higher order aberrations;
(b) measuring ocular aberrations generated by irregularities of the corneal topography;
(c) calculating the back surface design;
(d) calculating the back surface correction needed in terms of Zernike coefficients;
(e) converting the correction of (d) using the Visual Performance Detrimental Factor;
(f) calculating the residual aberrations;
(g) calculating the front surface correction needed in terms of Zernike coefficients;
(h) converting the correction of (g) using the Visual Performance Detrimental Factor;
(i) obtaining the relevant higher order aberrations for correction; and
(j) obtaining an optimised design for the front and back surface of the lens.

The corneal topography which will illustrate irregularities of the front surface of the cornea can be measured using any method.

The calculation of the back surface design may be carried out with an assumption that the corneal aberrations are reduced to zero. In one alternative, the calculation may assume that the back surface of the lens creates new aberrations or that there are still further aberrations from the corneal surface.

The calculation of the residual aberrations in step (g) may be the total minus the corneal aberrations or in one alternative may be a calculation taking the back surface aberrations into account.

The methods of the present invention may be further customised to take account of the actual subject's pupil size under determined lighting condition, usually low luminance.

In a further modification, the methods may include fitting the subject with a trial contact lens, measuring contact lens decentration and then compensating accordingly. In particular, the aberrations produced by the absence of coaxiality between the contact lens and the pupil of the eye may be considered. The trial contact lens may be of a similar design to a contact lens which will be subsequently prescribed. The lens may include correction for defocus to match the patient's requirements or a standard lens may be used. The trial lens is preferably allowed to equilibrate before the decentration is measured. Measurement of contact lens decentration and the subsequent compensation may be carried out by any suitable method. One example of a suitable method is described in US 6449843.

Lenses designed according to the methods of the present invention may be produced to correct the relative visual effect of different types of aberrations in which the visual performance detrimental factor has been considered. The lens may be a contact lens, an inlay, an onlay or an intra-ocular lens but is preferably a gas permeable contact lens. The lens may have a spherical or aspherical back surface. The lenses will be produced by any suitable method. Suitable methods include laser ablation, lathing, cast-moulding or machining.

Lenses designed according to the methods of the present invention may optimise the higher order aberration correction by producing the inverse aberration to the population mean aberration for rotationally symmetrical aberrations of third to tenth orders, most particularly the fourth to sixth orders. (Spherical Aberration: Z12 $Z_4^0$; and Z24 $Z_6^0$). The benefits of such designs include optimising the optical correction for the population average without changing the contact lens fitting technique for population using rotationally symmetrical contact lens and maintaining the comfort achieved by current "spherical" contact lenses.

Lenses designed according to the methods of the present invention may be rigid contact lenses. Rigid lenses maintain their shape without support and either do not deform when positioned in the eye or deform by a minimum amount. Rigid contact lenses are particularly useful in the correction of myopia and hyperopia particularly where significant levels of astigmatism are present.

Lenses designed according to the methods of the present invention may optimise the higher order aberration correction by producing the inverse aberration to the population mean aberration for rotationally and non-rotationally symmetrical aberrations of third to tenth orders, most particularly the fourth to sixth orders. (Coma Z7 $Z_3^{-1}$ and Z8 $Z_3^{+1}$, Secondary astigmatism Z11 $Z_4^2$ and Z13 $Z_4^2$, Spherical Aberration: Z12 $Z_4^0$; and Z23 $Z_6^{-2}$ and Z25 $Z_6^{+2}$ Z24 $Z_6^0$). The benefits of such designs include optimising the optical correction for the population average for populations currently using "spherical" and "toric" contact lenses, for those using toric contact lenses the result is achieved without changing the contact lens fitting technique and the comfort achieved by current toric contact lenses is maintained.

In a specific arrangement for "toric" contact lenses, the correction of higher order non rotationally symmetrical aberrations for the average population is particularly recommended as such rotationally symmetrical aberrations are correlated to the astigmatism present and greater than for populations with low levels of astigmatism and habitually corrected by "spherical" contact lenses.

The back surface of the contact lens may be spherical such that it is the front surface of the lens which is designed to achieve the targeted correction. In aspects of the present invention, the back surface may be toric. Toric contact lenses are usually used to correct astigmatism at least equal to 0.75 dioptre. In an alternative arrangement the back surface of the lens may be multi-spherical or multi-toric to achieve the desired fitting characteristics.

In an alternative modification, the back surface of the lens may be designed and/or formed to neutralise the mean corneal rotationally symmetrical aberrations. In this arrangement the front surface is designed to achieve the desired correction. This alternative modification is particularly useful for soft contact lenses which change shape when placed on the eye. The change in shape depends upon the mechanical properties of the lens which are influenced by the rigidity of the contact lens material and the profile of the lens and the relative geometry of the contact lens back surface and corneal front surface. Matching the mean corneal front surface rotationally symmetrical aberrations minimises the effect of the shape of the lens on the eye.

In a still further alternative modification, the back surface of the lens may be designed to optimise the mechanical fit of the lens. In this arrangement the front surface is designed to achieve the targeted correction. Spherical or non-spherical surfaces such as aspheric surfaces, rotationally symmetrical surfaces such as certain polynomial progressions or other continuous or non-continuous surfaces may be used. For the second alternative arrangement the spherical, multi-spherical and multi-non-spherical surfaces may be used alone or in combination.

In this connection it should be noted that if a good visual performance is to be achieved, the lens should have a good mechanical fit. This is particularly important where aspheric surfaces are present. It is well known in the art that in order to achieve optimal fit it may be necessary to modify the contact lens back surface which may lead to its shape not matching the front surface of the cornea.

Whichever design is selected, the design may be custom made or may be suitable for the whole population or in one alternative, a series of designs may be provided to optimise the results for sub-populations based on the ocular, such as corneal topography, and/or refractive characteristics and/or for demographics, such as age.

In one arrangement, rotationally symmetrical contact lens design is provided which achieves improved optical results by incorporating the correction of Z12 and Z24 aberrations and possibly all higher order rotationally symmetrical aberrations.

The rotationally symmetrical aberrations are preferably correlated to the refractive error. The mean rotationally symmetrical aberration is different for different spherical refractive error, in particular for high myopic corrections. In a second arrangement, a mean correction of rotationally symmetrical aberrations that would differ for different prescriptions may be incorporated in the design.

The non rotationally symmetrical aberrations are preferably correlated to the cylindrical refractive error. In particular, for higher cylinders (>1.25D) the mean of these aberrations is significantly greater than for low cylinders. In a third arrangement, a mean correction of not rotationally symmetrical aberrations that would differ with different cylindrical prescriptions is incorporated in the design.

For simultaneous vision bifocal contact lenses, it is generally essential to optimise the quality of both the distance and near images. In a fourth arrangement, the correction of aberrations as suggested in second and third arrangement can be incorporated in such designs. This correction is applicable to concentric type bifocal in particular multi-ring bifocal.

Further, one of the higher order aberrations, spherical aberration Z12, has been shown to increase with age. In a further arrangement, a different level of aberration correction can be incorporated in the designs for presbyopes compared to non-prebyopes younger population. Such consideration is particularly suitable for the design of bifocal contact lenses.

In a fifth arrangement, different levels of higher order aberrations correction can be incorporated into a bifocal contact lens (to correct presbyopia) for early to medium presbyopes which are generally of up to 55 years of age or having up to +1.75D addition and for established presbyopes which are generally over 55 years of age or have an addition of +2.00D and above.

In a sixth arrangement, different levels of higher order aberration correction can be incorporated in rotationally symmetrical bifocal contact lens designs (to correct presbyopia) for early to medium presbyopes which are generally of up to 55 years of age or having up to +1.75D addition and for established presbyopes which are generally over 55 years of age or have an addition of +2.00D and above.

In a seventh arrangement, a rotationally symmetrical bifocal can be provided in which the correction of spherical aberration (e.g. $Z_4^0$ (Z12)), which is achievable without need for rotational stabilisation will be of a greater magnitude for established presbyopes (eg: over 55 years of age or +2.00 addition or above) than for early to medium presbyopes (eg: up to 55 years of age or up to +1.75D addition).

In an eighth arrangement, the determination of the level of rotationally symmetrical aberrations to correct for rotationally symmetrical single vision contact lenses for an average population needs to be measured with a population of up to 55 years old to match the usual contact lenses population demographics.

In a ninth arrangement, different levels of overall higher order aberrations correction will be incorporated in non rotationally symmetrical bifocal contact lenses designs (to correct presbyopia) for early to medium presbyopes (up to 55 years oldof age orup to+1.75D addition) and for established presbyopes (over 55 years of age or addition +2.00D and above).

In a tenth arrangement, a non rotationally symmetrical bifocal can be provided in which the correction of aberrations, in particular $Z_3^{-1}$ (Z7), will be of a greater magnitude for established presbyopes (eg: over 55years of age or +2.00 addition or above) than for early to medium presbyopes (eg: up to 55 years of age or up to +1.75D addition).

In an eleventh arrangement, the determination of the level of rotationally and non rotationally symmetrical aberrations to correct for non rotationally symmetrical single vision contact lenses for an average population is determined with a population of up to 55 years old to match the usual contact lenses population demographics.

In a twelfth arrangement, different mean level of aberration corrections is incorporated into a lens for a different range of corrections to optimize optical performance. In particular, different levels of rotationally symmetrical aberration corrections are incorporated in the design of symmetrical contact lenses.

When producing the lenses designed according to the methods of the present invention it may be desirable to take the moulding of the lens on the average front surface of the cornea into consideration. Any suitable level of moulding may be taken into consideration including: no moulding in the case of a very rigid lens; total moulding in the case of a very flexible lens; and partial moulding in the case of soft lenses with intermediate moulding characteristics.

In order to further optimise the design of the lens, it may be desirable to carry out an in vitro trial. The trial will comprise the steps of

(a) constructing a lens of the fifth aspect of the invention;
(b) placing the lens on a reference corneal surface;
(c) measuring the front surface of the lens on the preference corneal surface;
(d) calculating the true moulding for the lens; and
(e) calculating the modified design from the data.

The reference corneal surface may be produced from any suitable material including plastics such as Perspex, glass, or other rigid or semi-rigid materials.

The steps of the trial can be repeated as often as is necessary until the design is optimised.

Additionally or alternatively the design of the lens may be optimised using an in vivo clinical trial. The clinical trial will comprise the steps of:

(a) constructing a lens of the fifth aspect of the invention;
(b) selecting a test population representative of the mean of the target or targets populations;
(c) measuring the front surface of the lens fitted on the test population;
(f) calculating the true moulding for the lens; and
(d) calculating the modified design from the data.

The steps of the trial can be repeated as often as is necessary until the design is optimised.

Whether the in vivo or in vitro optimisation trials are used, or both, the front surface of the lens may be measured by any suitable techniques. Suitable techniques include interferometry.

The lens designed according to the methods of the present invention may include any of the conventional lens design features such as those used to achieve lens stabilisation on the eye. Such lens design features include, but are no limited to, prism ballast, truncation, peripheral thinning, slab off, double slab off. One or more of these features may be present.

The present invention will now be described for exemplification purposes only with reference to the following examples and figures in which:

Figure 1    is a graphic representation of the Mean +- 2SE for RMSHO, RMS3, RMS4, RMS5, RMS 6 and RM124 of Table 1;

Figure 2    is a graphic representation of the Mean +- 2SE for Z12, Z24, Z7, Z8, Z11 and Z13 of Table 1;

Figure 3    is a graphic representation of the Mean +- 2SE for RMSHO, RMS3, RMS4, RMS5, RMS 6 and RM124 of Table 2;

Figure 4    is a graphic representation of the Mean +- 2SE for Z12, Z24, Z7, Z8, Z11 and Z13 of Table 2;

Figure 5    is a graphic representation of the Mean +- 2SE for RMSHO, RMS3, RMS4, RMS5, RMS 6 and RM124 of Table 3; and

Figure 6    is a graphic representation of the Mean +- 2SE for Z12, Z24, Z7, Z8, Z11 and Z13 of Table 3.

## Example 1

The objective of the example was to assess the effects of specific higher order aberrations on visual performance and compare it to the effects of spherical defocus. In the example, distorted visual acuity charts were generated for each specific aberration corresponding to a specific Zernike coefficient. The visual performance measured with those charts was compared to that of spherical defocus as it is the defect commonly corrected by spectacles or contact lenses. All charts were blurred by the same total amount of aberrations.

The baseline visual performance was determined with best corrected sphero-cylindrical refraction and was used as a reference to assess the visual loss due to defocus and higher order aberrations. Calculations of VPDF are shown for a 6 mm pupil.

|  | Visual Performance - High Contrast | Visual Loss - High Contrast | Visual Performance - Low Contrast | Visual Loss - Low Contrast |
|---|---|---|---|---|
| Optimal | +1.0 |  | -1.0 |  |
| Defocus 0.25D | -1.6 | -2.6 | -2.7 | -1.7 |
| Defocus 0.50D | -4.2 | -5.2 | -5.2 | -4.2 |
| Defocus 0.75D | -5.4 | -6.4 | -6.8 | -5.8 |

Data given in VA unit = -10 LogMAR (0=20/20, positive values >20/20; negative values <20/20) 1VA unit = 1 VAline

The mean visual loss for a 6 mm pupil for high and low contrast letters for a defocus of 0.5 diopters was -4.7 VA lines as calculated by (-5.2 for high contrast + -4.2 for low contrast)/2.

|  | Visual Performance High Contrast | Visual Loss High Contrast | Visual Performance Low Contrast | Visual Loss Low Contrast | Mean Visual Loss | Correcting Factor |
|---|---|---|---|---|---|---|
| Defocus $Z_2^0$ | -4.62 | -5.62 | -5.54 | -4.54 | -5.08 | 1.1 |
| Secondary Astigmatism $Z_4^{-2}$ | -4.56 | -5.56 | -5.44 | -4.44 | -5.00 | 1.1 |
| Secondary Astigmatism $z_4^2$ | -4.18 | -5.18 | -5.17 | -4.17 | -4.67 | 1 |
| Coma $Z_3^1$ | -2.42 | -3.42 | -4.34 | -3.34 | -3.38 | 0.7 |
| Coma $Z_3^{-1}$ | -2.36 | -3.36 | -4.38 | -3.38 | -3.37 | 0.7 |
| Spherical Aberration $Z_4^0$ | -2.12 | -3.12 | -5.33 | -4.33 | -3.72 | 0.8 |

(continued)

|  | Visual Performance High Contrast | Visual Loss High Contrast | Visual Performance Low Contrast | Visual Loss Low Contrast | Mean Visual Loss | Correcting Factor |
|---|---|---|---|---|---|---|
| Trefoil $Z_3^{-3}$ | -0.63 | -1.63 | -3.66 | -2.66 | -2.14 | 0.5 |
| Trefoil $Z_3^3$ | -0.50 | -1.50 | -3.83 | -2.83 | -2.16 | 0.5 |
| Tetrafoil $Z_4^{-4}$ | -0.16 | -1.16 | -2.79 | -1.79 | -1.47 | 0.3 |
| Tetrafoil $Z_4^4$ | -0.06 | -1.06 | -2.72 | -1.72 | -1.39 | 0.3 |

The RMS value for higher order aberrations is normally calculated as follows:

$$RMS = \text{sq root} \left( (Z_4^{-2})^2 + (Z_4^2)^2 + (Z_3^1)^2 + (Z_3^{-1})^2 + (Z_4^0)^2 + (Z_3^{-3})^2 + (Z_3^3)^2 + (Z_4^{-4})^2 + (Z_4^4)^2 \right)$$

The RMS corrected for visual factor will vary depending on the pupil size. For the example of a 6 mm pupil size, the RMS will be calculated as follows:

$$RMS = \text{sq root} \left( 1.1*(Z_4^{-2})^2 + 1.1*(Z_4^2)^2 + 0.7*(Z_3^1)^2 + 0.7(Z_3^{-1})^2 + 0.8*(Z_4^0)^2 + 0.5*(Z_3^{-3})^2 + \right.$$
$$\left. 0.5*(Z_3^3)^2 + 0.3(Z_4^{-4})^2 + 0.3*(Z_4^4)^2 \right)$$

For different pupil size, the correcting factor will be different and therefore the RMS calculation will differ.

By comparing the effect of specific higher order aberrations on visual performance, it was found that different types of higher order aberrations affected visual performance differently.

It was noted that each individual Zernike term produced a different effect on visual performance. The relative effect of each individual Zernike term did not vary with pupil size or the contrast of letters such that coefficients affecting visual performance the most or the least remained the same independently of the test conditions. At all pupil sizes and for high and low contrast letters, fourth order secondary astigmatism terms affected visual performance the least and less than spherical defocus.

In general, Zernike terms on the centre of the Zernike pyramid tended to affect visual performance more than terms on the edge of the pyramid. For third order terms, coma had a more important detrimental effect than trefoil terms and for fourth order terms spherical aberration and secondary astigmatism degraded visual performance more than trefoil terns.

Spherical aberration tended to have a more important detrimental effect on low contrast letters.

As the pupil enlarged, the specific effect of each aberration on visual performance became more obvious, especially for high contrast letters. The differences between high order effects of specific types of aberrations on visual performance were the highest (approximately 5VA lines on high contrast charts and approximately 3 VA lines on low contrast letters) for low luminance i.e. for larger pupils and thus for significantly higher amounts of higher order aberrations.

**Example 2**

Details of the experimental data to support the premises of the present invention are set out below.

OVERALL POPULATION

Data

Three sets of data are given: i for the overall population; ii for the population with astigmatism 0.00 to 0.75D, conventionally corrected with a rotationally symmetrical soft contact lens (e.g. spherical contact lens); and iii for the population astigmatism greater than 0.75D corrected with a non rotationally symmetrical soft contact lens (e.g. toric soft contact lens).

For each variable the mean value and standard error of the mean are given in a tabular form. The 95% confidence interval is also given, in a graphical form for the same variables.

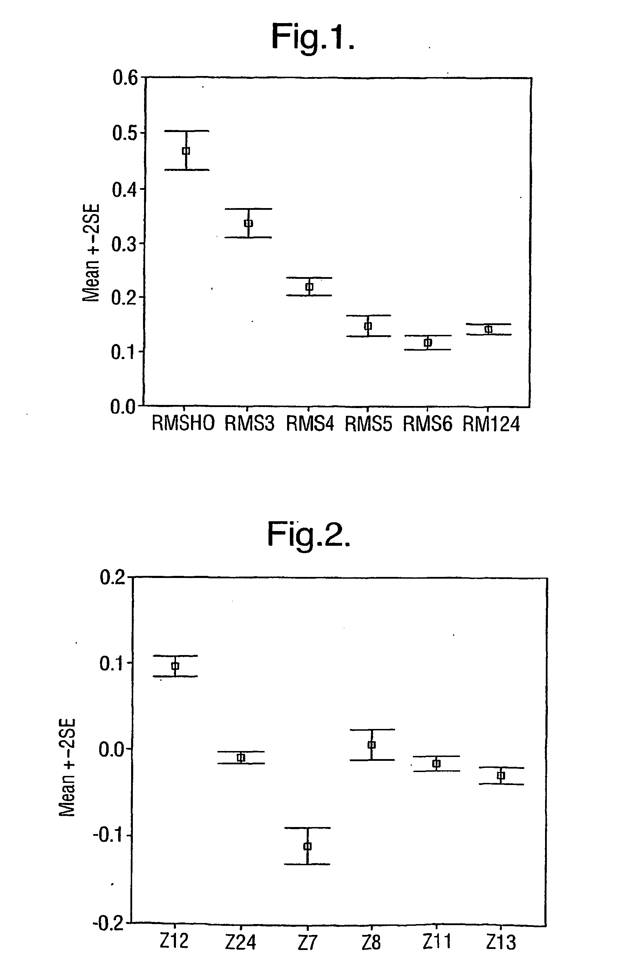

Descriptives - Overall population (All cylinder powers) see Table 1 and Figures 1 and 2.

Table 1

| LUM | | N | Mean | |
|---|---|---|---|---|
| | | Statistic | Statistic | Std. Error |
| 3.00 | RMS HO (higher order) | 424 | .469 | .018 |
| | RMS 3 | 424 | .338 | .013 |
| | RMS 4 | 424 | .220 | .008 |
| | RMS 5 | 424 | .149 | .009 |
| | RMS 6 | 424 | .117 | .006 |
| | z12o | 424 | .096 | .006 |
| | z24o | 424 | -.009 | .003 |
| | z7o | 424 | -.110 | .010 |
| | Z8O | 424 | .006 | .009 |
| | Z11O | 424 | -.016 | .004 |
| | z13o | 424 | -.029 | .005 |
| | RMS 12&24 | 424 | .143 | .005 |

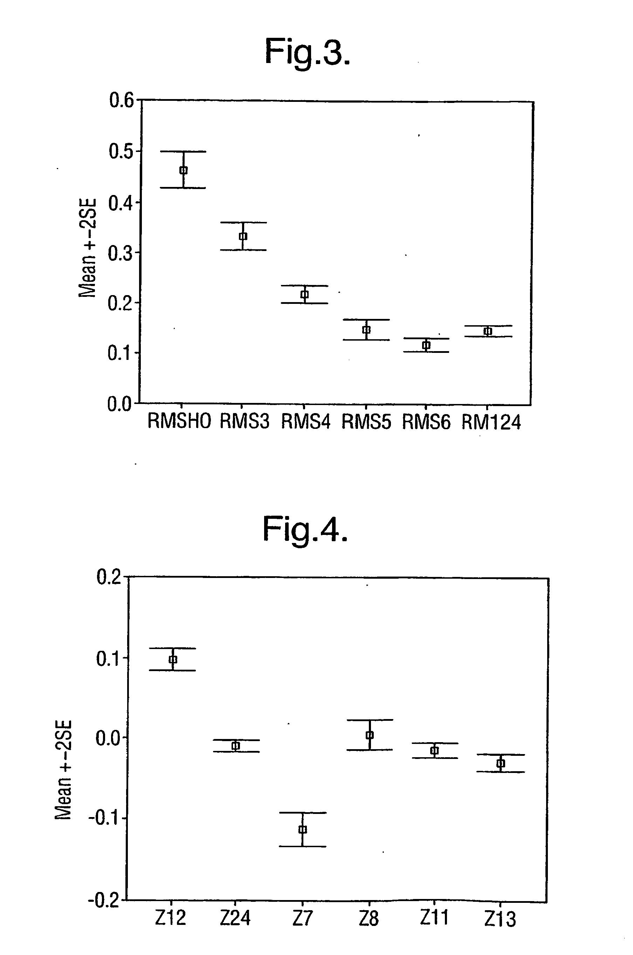

Descriptives - Cylinder up to 0.75 D - see Table 2 and Figures 3 and 4

Table 2

| | | N | Mean | |
|---|---|---|---|---|
| | | Statistic | Statistic | Std. Error |
| | RMSHO | 372 | .464 | .018 |
| | RMS3 | 372 | .333 | .014 |
| | RMS4 | 372 | .218 | .009 |
| | RMS5 | 372 | .147 | .010 |
| | RMS6 | 372 | .117 | .007 |
| | z12 | 372 | .098 | .007 |
| | z24 | 372 | -.010 | .003 |
| | z7 | 372 | -.113 | .011 |
| | Z8 | 372 | .004 | .009 |
| | Z11 | 372 | -.015 | .005 |
| | z13 | 372 | -.030 | .005 |
| | RM124 | 372 | .145 | .005 |
| | Valid N (listwise) | 372 | | |

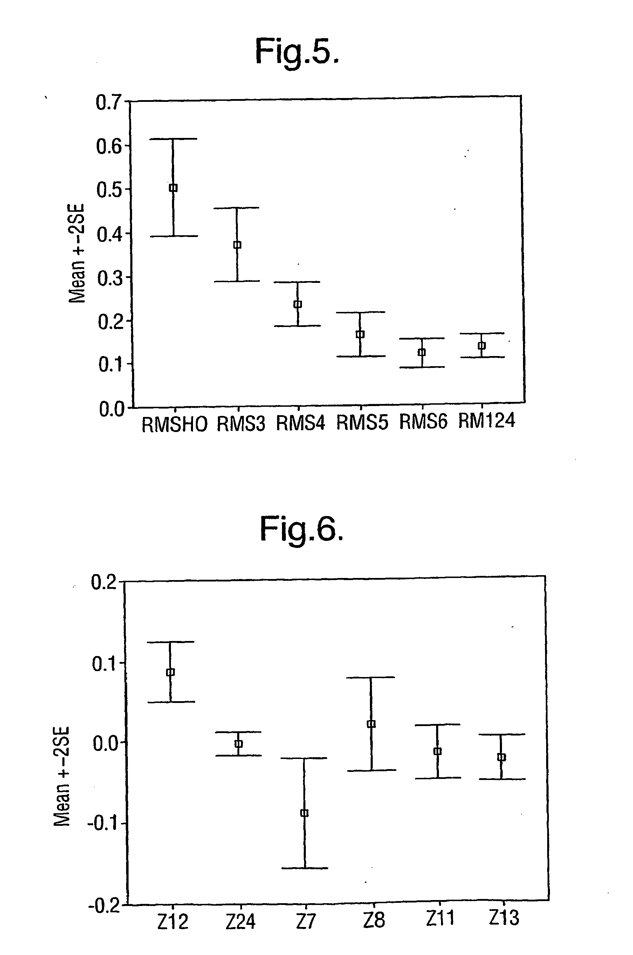

Descriptives - Cylinder >0.75D - see Table 3 and Figures 5 and 6

Table 3

| | | N | Mean | |
|---|---|---|---|---|
| | | Statistic | Statistic | Std. Error |
| | RMSHO | 52 | .504 | .055 |
| | RMS3 | 52 | .373 | .042 |
| | RMS4 | 52 | .234 | .025 |
| | RMS5 | 52 | .163 | .026 |
| | RMS6 | 52 | .118 | .017 |
| | z12 | 52 | .087 | .018 |
| | z24 | 52 | -.002 | .007 |
| | z7 | 52 | -.088 | .034 |
| | Z8 | 52 | .020 | .029 |
| | Z11 | 52 | -.016 | .016 |
| | z13 | 52 | -.023 | .014 |
| | RM124 | 52 | .133 | .014 |
| | Valid N (listwise) | 52 | | |

AGE GROUP EFFECT

Data

The data is given for three different age groups: i) non presbyopes (<45 years); ii) early to medium presbyopes (45 to 55 years); iii) established presbyopes (>55 years).

Three sets of data are given: i) for the overall population; ii) for the population with astigmatism from 0.00 to 0.75D, conventionally corrected with a rotationally symmetrical soft contact lens (eg: Spherical contact lens); iii) for the population corrected with a non symmetrical rotational soft contact lens (eg: Toric contact lens).

AGE - Overall Population (All cylinder powers) - see Table 4

Table 4

| Z12 p=0.038 | <45 Yrs | >55 Yrs | 45-55 Yrs |
|---|---|---|---|
| Mean SNK (5%) | 0.084 | 0.112 | 0.120 |

| Z8 p=0.002 | >55 Yrs | 45-55 Yrs | <45 Yrs |
|---|---|---|---|
| Mean SNK (5%) | -0.052 | -0.013 | 0.029 |

AGE - Cylinder up to 0.75D - see Table 5

### Table 5

| Z12<br>p=0.028 | <45 Yrs | 45-55 Yrs | >55 Yrs |
|---|---|---|---|
| Mean<br>SNK (5%) | 0.085 | 0.118 | 0.126 |
| | | | |
| Z8<br>p=0.005 | >55 Yrs | 45-55 Yrs | <45 Years |
| Mean<br>SNK (5%) | -0.060 | -0.011 | +0.024 |

AGE - Cylinder >0.75D - see Table 6

### Table 6

| Z7<br>p=0.028 | >55 Yrs | 45-55 Yrs | <45 Yrs |
|---|---|---|---|
| Mean<br>SNK (5%) | -0.207 | -0.050 | -0.008 |

REFRACTIVE ERROR

Data

The data is given for five different refractive groups: i. Myopes -6.0 and above; ii. Myopes -3.0 to -6.00; iii. Myopes -0.50 to -3.00; iv. Emmetrope -0.25 to <0.75; v. Hyperopes +0.75 and above.

Three sets of data are given: i) for the overall population; ii) for the population with astigmatism from 0.00 to 0.75D, conventionally corrected with a rotationally symmetrical contact lens (eg: Spherical contact lens); iii) for the population corrected with a non symmetrical rotational contact lens (eg: Toric contact lens).

Refraction Groups - Overall population (All cylinder powers) - see Table 7

## Table 7

| RMS TOTAL p=0.014 | HYP | -3.0→-6.0 | -0.50→-3.00 | EMM | <-6.0 |
|---|---|---|---|---|---|
| Mean SNK (5%) | 0.376 | 0.426 | 0.516 | 0.530 | 0.586 |

| RMS 3 p=0.045 | HYP | -3.0→-6.0 | -0.50→-3.00 | EMM | <-6.0 |
|---|---|---|---|---|---|
| Mean SNK (5%) | 0.277 | 0.312 | 0.359 | 0.396 | 0.411 |

| RMS 5-6 p=0.002 | HYP | -3.0→ -6.0 | EMM | -0.50 → -3.0 | <-6.0 |
|---|---|---|---|---|---|
| Mean SNK (5%) | 0.120 | 0.166 | 0.225 | 0.238 | 0.250 |

| Z12 p=0.006 | -0.50 → -3.0 | -3.0→ -6.0 | <-6.0 | EMM | HYP |
|---|---|---|---|---|---|
| Mean SNK (5%) | 0.080 | 0.081 | 0.112 | 0.115 | 0.144 |

| Z8 p=0.019 | HYP | -0.50 → -3.0 | -3.0→ -6.0 | EMM | -6.0< |
|---|---|---|---|---|---|
| Mean SNK (5%) | -0.062 | 0.001 | 0.018 | 0.024 | 0.055 |

| Z24 P=0.003 | -0.50 → -3.0 | EMM | HYP | -3.0→ -6.0 | -6.0< |
|---|---|---|---|---|---|
| Mean SNK (5%) | -0.025 | -0.018 | -0.003 | 0.003 | 0.006 |

Refraction groups- Cylinder up to 0.75D - see Table 8

## Table 8

| RMS TOTAL p=0.013 | HYP | -3.0→ -6.0 | -0.50 → -3.0 | EMM | <-6.0 |
|---|---|---|---|---|---|
| Mean SNK (5%) | 0.386 | 0.407 | 0.522 | 0.524 | 0.597 |

| RMS 3 p=0.042 | HYP | -3.0→ -6.0 | -0.50 → -3.0 | EMM | <-6.0 |
|---|---|---|---|---|---|
| Mean SNK (5%) | 0.281 | 0.296 | 0.364 | 0.394 | 0.403 |

| RMS 5-6 p=0.003 | HYP | -3.0→-6.0 | EMM | -0.5→-3.0 | <-6.0 |
|---|---|---|---|---|---|
| Mean SNK (5%) | 0.124 | 0.159 | 0.212 | 0.241 | 0.267 |

| Z12 p<0.001 | -0.50 → -3.0 | -3.0→-6.0 | EMM | HYP | <-6.0 |
|---|---|---|---|---|---|
| Mean SNK (5%) | 0.076 | 0.081 | 0.117 | 0.153 | 0.159 |

| Z24 p=0.010 | -0.50 → -3.0 | EMM | HYP | -6.0< | -3.0→-6.0 |
|---|---|---|---|---|---|
| Mean SNK (5%) | -0.026 | -0.016 | -0.006 | 0.002 | 0.003 |

| Z7 p=0.027 | -6.0< | EMM | -0.50 → -3.0 | -3.0 → -6.0 | HYP |
|---|---|---|---|---|---|
| Mean SNK (5%) | -0.219 | -0.147 | -0.135 | -0.083 | -0.080 |

| Z13 p=0.002 | EMM | -6.0< | -0.50 → -3.0 | -3.0 → -6.0 | HYP |
|---|---|---|---|---|---|
| Mean SNK (5%) | -0.084 | -0.043 | -0.025 | -0.021 | -0.015 |

Refraction groups - Cylinder > 0.75D - No significant differences were observed.

For all cylinders for the overall population, none of the RMS of the higher order aberrations 95% confidence intervals included zero, demonstrating that for the average of the population these aberrations are significant. Thus correction of the higher aberrations quantified here will produce an improved optical characteristic for the overall population.

For the cylinder up to 0.75D, none of the RMS of the higher order aberrations 95% confidence intervals included zero, demonstrating that for the average of the population these aberrations are significant. Thus it can be seen that rotationally symmetrical contact lens designs will achieve improved optical results by incorporating the correction ofZ 12 and Z24 aberrations and possibly all higher order rotationally symmetrical aberrations.

For cylinder > 0.75D, none of the RMS of the higher order 95% confidence intervals included zero, demonstrating that for the average of the population these aberrations are significant. Thus rotationally stabilised contact lens designs will achieve improved optical results for the population by incorporating the correction of the higher order aberrations quantified here.

The age group effect was then considered.

For all cylinders:

Different levels of overall higher order aberrations correction will be incorporated in bifocal contact lens designs (to correct presbyopia) for early to medium presbyopes (up to 55 years old of age or up to +1.75D addition) and for established presbyopes (over 55 years of age or addition +2.00D and above).

The overall comparisons of the individual Zernike coefficients were significant for $Z_3^{+1}$ (Z8) at low luminance (p=0.002) and $Z_4^0$ (Z12) (p=0.038).

The individual comparisons for the two Zernike coefficients $Z_3^1$ and $Z_4^0$ revealed higher magnitude for the older age groups.

For the cylinder ≤ 075D

Different levels of overall higher order aberrations correction can be incorporated in rotationally symmetrical bifocal contact lens designs (to correct presbyopia) for early to medium presbyopes (up to 55 years old of age or up to +1.75D addition) and for established presbyopes (over 55 years of age or addition +2.00D and above).

The overall comparisons of the individual Zemike coefficients were significant for $Z_3{}^{+1}$ (Z8) at low luminance (p=0.005) and $Z_4{}^0$ (Z12) (p=0.028).

The individual comparisons for both Zernike coefficient revealed higher magnitude for the two presbyopic groups compared to the non presbyope group for $Z_3{}^{-1}$ (Z7) and $Z_4{}^0$ (Z12) at low luminance.

Thus in rotationally symmetrical bifocal, the correction of $Z_4{}^0$ (Z 12) which is achievable without need for rotational stabilisation will be of a greater magnitude for established presbyopes (eg: over 55 years of age or +2.00 addition or above) than for early to medium presbyopes (eg: up to 55 years of age or up to +1.75D addition).

Further the determination of the level of rotationally symmetrical aberrations to correct for rotationally symmetrical single vision contact lenses for an average population needs to be measured with a population of up to 55 years old to match the usual contact lenses population demographics.

For cylinder>0.75D

Different levels of overall higher order aberrations correction will be incorporated in non rotationally symmetrical bifocal contact lenses designs (to correct presbyopia) for early to medium presbyopes (up to 55 years old of age or up to +1.75D addition) and for established presbyopes (over 55 years of age or addition +2.00D and above).

The overall comparisons of the individual Zemike coefficients were significant for $Z_3{}^{-1}$ (Z7) at low luminance (p=0.028).

The individual comparisons for Zemike $Z_3{}^{-1}$ (Z7) coefficient revealed statistically significant higher coefficient for the older age group than for the younger groups for $Z_3{}^{-1}$ (Z7) at low luminance.

Thus in non rotationally symmetrical bifocal, the correction of aberrations, in particular $Z_3{}^{-1}$ (Z7) will be of a greater magnitude for established presbyopes (e.g.: over 55 years of age or +2.00 addition or above) than for early to medium presbyopes (e.g.: up to 55 years of age or up to +1.75D addition).

Further the determination of the level of rotationally and non rotationally symmetrical aberrations to correct for non rotationally symmetrical single vision contact lenses for an average population needs to be determined with a population of up to 55 years old to match the usual contact lenses population demographics.

Refractive error was then considered.

For all cylinders:

i. The overall comparison revealed significant differences between corrections for the overall higher aberrations (p=0.014), the third (p=0.045) and combined fifth and sixth (p=0.002) order aberrations.

Individual comparisons between the different groups showed that the overall higher order; third and fifth and sixth order aberrations were lower for the hyperopic group than for the high myopes.

Thus different mean level of aberration corrections can be incorporated for different ranges of corrections to optimise optical performance.

ii. The Zemike coefficient Z8(p=0.019), Z12 (p=0.006) & Z24(p=0.003) were overall significant for the refractive group.

The cylinder up to 0.75D:

i. The overall comparison revealed significant differences between corrections for the overall higher aberrations (p=0.013), the third (p=0.042) and combined fifth and sixth (p=0.003) order aberrations.

Individual comparisons between the different groups showed that the overall higher order; third and fifth and sixth order aberrations were lower for the hyperopic group than for the high myopes.

ii. The Zemike coefficient Z7 (p=0.019), Z12 (p<0.001), Z13 (p=0.002), Z24 (p=0.0 10) and Z7 (p=0.027) were overall significant for the refractive group.

Thus different level of rotationally symmetrical will be incorporated in the design of rotationally symmetrical contact lenses.

**Claims**

1. A method for designing a custom lens having a spherical back surface which is tailored for the relative visual effect of different types of aberrations comprising the steps of:

   (a) measuring total ocular higher order aberrations for a given pupil and for specific pupil sizes;
   (b) calculating the front surface correction needed in terms of Zemike coefficients;

(c) converting the correction using the Visual Performance Detrimental Factor, wherein the Visual Performance Detrimental Factor is calculated using the following steps:

- Calculation of visual acuity loss compared to baseline performance, which is the best corrected visual acuity, for high contrast and low contrast letters, wherein the best corrected visual acuity is determined with best correct sphero-cylindrical refraction, visual acuity loss = best corrected visual acuity - visual performance measured
- Calculation of the mean visual acuity loss for high and low contrast charts

$$\text{mean visual acuity loss} = ((\text{visual acuity loss high contrast}) + (\text{visual acuity loss low contrast})) / 2$$

- Calculation of the mean visual acuity loss for all the individual Zernike coefficients
- Calculation of the VPDF for each individual Zemike coefficient VPDF (Zx) = (mean visual acuity loss for Zx) / (mean visual acuity loss for defocus);

(d) obtaining the relevant higher order aberrations for correction; and
(e) obtaining the optimised design for the front surface of the lens.

2. A method in which the VPDF is used to optimise the design of both the front and back surface of the lens comprising the steps of:

(a) measuring total ocular higher order aberrations for a given pupil and for specific pupil sizes;
(b) measuring ocular aberrations generated by irregularities of the corneal topography;
(c) calculating the back surface design;
(d) calculating the back surface correction needed in terms of Zemike coefficients;
(e) converting the correction of (d) using the Visual Performance Detrimental Factor, wherein the Visual Performance Detrimental Factor is calculated using the following steps:

- Calculation of visual acuity loss compared to baseline performance, which is the best corrected visual acuity, for high contrast and low contrast letters, wherein the best corrected visual acuity is determined with best corrected sphero-cylindrical refraction,

$$\text{visual acuity loss} = \text{best corrected visual acuity} - \text{visual performance measured}$$

- Calculation of the mean visual acuity loss for high and low contrast charts

$$\text{mean visual acuity loss} = ((\text{visual acuity loss high contrast}) + (\text{visual acuity loss low contrast})) / 2$$

- Calculation of the mean visual acuity loss for all the individual Zemike coefficients
- Calculation of the VPDF for each individual Zemike coefficient VPDF (Zx) = (mean visual acuity loss for Zx) / (mean visual acuity loss for defocus);

(f) calculating the residual aberrations;
(g) calculating the front surface correction needed in terms of Zemike coefficients;
(h) converting the correction of (g) using the Visual Performance Detrimental Factor, wherein the Visual Performance Detrimental Factor is calculated as described in step (e) above;
(i) obtaining the relevant higher order aberrations for correction; and
(j) obtaining an optimised design for the front and back surface of the lens.

3. The method according to Claim 1 or 2 wherein the custom lens may be a contact lens, an inlay, an onlay or an intra-ocular lens.

4. The method according to Claim 3 wherein the custom lens is a soft or rigid contact lens.

5. The method according to any one of Claims 1 to 4 wherein the total ocular higher order aberrations are measured using a wavefront sensor.

6. The method according to Claim 1 wherein the calculation of the back surface design is carried out with an assumption that the corneal aberrations are reduced to zero.

7. The method according to Claim 1 wherein the calculation assumes that the back surface of the lens creates new aberrations or that there are still further aberrations from the corneal surface.

8. The method according to Claim 2 wherein the calculation of the residual aberrations in step (g) is the total minus the corneal aberrations.

9. The method according to Claim 2 wherein the calculation of the residual aberrations in step (g) takes into account the back surface aberrations.

10. A method according to any one of Claims I to 9 wherein the method is further customised to take account of the subject's pupil size.

11. A method according to any one of Claims 1 to 10 additionally comprising the steps of fitting the subject with a trial contact lens, measuring contact lens decentration and then compensating accordingly.

12. A method according to Claim 11 wherein the aberrations produced in the absence of coaxiality between the contact lens and the pupil of the eye is considered.

13. The method of Claim 1 or 2, wherein the step of calculating visual acuity loss compared to baseline performance comprises the steps of:

   (a) showing at least one vision test chart to a test subject, wherein the test chart comprises images and is suitable for measuring the visual effects of single or groups of higher order aberrations wherein the images are distorted with single or groups of higher order aberrations of known optical effects and the readability of the test chart provides an indication of the visual effect of the optical effects;
   (b) noting the visual effect of the distortions by recording the readability of the chart; and
   (c) comparing the relative readability of such distorted charts by a test panel of subjects who read or have read all the charts to provide an indication of the visual effect of the optical effects.

14. A method according to Claim 13 wherein the test chart is suitable for measuring the effects of higher order aberrations wherein the images are deformed with higher order aberrations of fixed optical effects.

15. A method according to Claim 14 wherein the images are distorted by defocus of different RMS values.

16. A method according to Claim 14 wherein the images are distorted by optical effects of different RMS values.

17. A method according to any one of Claims 1 to 16, wherein the method further comprises the step of producing a lens conforming to the optimised lens design.

**Patentansprüche**

1. Verfahren zum Gestalten einer kundenspezifischen Linse mit einer sphärischen Rückseite, die für den relativen visuellen effekt verschiedener Arten von Aberrationen maßgeschneidert ist, umfassend folgende Schritte:

   (a) Messen der gesamten okularen Aberrationen höherer Ordnung für eine gegebene Pupille und für bestimmte Pupillengrößen;
   (b) Berechnen der benötigten Vorderseitenkorrektur in Bezug auf Zernike-Koeffizienten;
   (c) Umwandeln der Korrektur unter Verwendung des die Sehleistung schädigenden Faktors (VPDF), wobei der die Sehleistung schädigende Faktor unter Verwendung der folgenden Schritte berechnet wird:

• Berechnen des Sehschärfeverlusts verglichen mit der Ausgangsleistung, bei der es sich um die beste korrigierte Sehschärfe handelt, für Buchstaben mit hohem Kontrast und niedrigem Kontrast, wobei die beste korrigierte Sehschärfe mit korrekter sphärozylindrischer Refraction bestimmt wird,

$$\text{Sehschärfeverlust} = \text{beste korrigierte Sehschärfe} - \text{gemessene Sehleistung}$$

• des mittleren Sehschärfeverlusts für Tafeln mit hohem und niedrigem Kontrast

$$\text{mittlerer Sehschärfeverlust} = ((\text{Sehschärfeverlust hoher Kontrast}) + (\text{Sehschärfeverlust niedriger Kontrast})) / 2$$

• Berechnen des mittleren Sehschärfeverlusts für alle einzelnen Zernike-Koeffizienten
• Berechnen des VPDF für jeden einzelnen Zernike-Koeffizienten

$$\text{VPDF (Zx)} = (\text{mittlerer Sehschärfeverlust für Zx}) / (\text{mittlerer Sehschärfeverlust für Defokussierung});$$

(d) Beschaffen der relevanten Aberrationen höherer Ordnung für die Korrektur; und
(e) Beschaffen der optimierten Gestalt für die Vorderseite der Linse.

2. Verfahren, bei dem der VPDF verwendet wird, um die Gestalt sowohl der Vorder- als auch der Rückseite der Linse zu optimieren, umfassend folgende Schritte:

(a) Messen der gesamten okularen Aberrationen höherer Ordnung für eine gegebene Pupille und für bestimmte Pupillengrößen;
(b) Messen von durch Ungleichmäßigkeiten der Hornhauttopographie erzeugten okularen Aberrationen;
(c) Berechnen der Gestalt der Rückseite;
(d) Berechnen der benötigten Rückseitenkorrektur in Bezug auf Zernike-Koeffizienten;
(e) Umwandeln der Korrektur aus (d) unter Verwendung des die Sehleistung schädigenden Faktors (VPDF), wobei der die Sehleistung schädigende Faktor unter Verwendung der folgenden Schritte berechnet wird:

• des Sehschärfeverlusts verglichen mit der Ausgangsleistung, bei der es sich um die beste korrigierte Sehschärfe handelt, für Buchstaben mit hohem Kontrast und niedrigem Kontrast, wobei die beste korrigierte Sehschärfe mit bester korrigierter sphärozylindrischer Refraktion bestimmt wird,

$$\text{Sehschärfeverlust} = \text{beste korrigierte Sehschärfe} - \text{gemessene Sehleistung}$$

• Berechnen des mittleren Sehschärfeverlusts für Tafeln mit hohem und niedrigem Kontrast

$$\text{mittlerer Sehschärfeverlust} = ((\text{Sehschärfeverlust hoher Kontrast}) + (\text{Sehschärfeverlust niedriger Kontrast})) / 2$$

• Berechnen des mittleren Sehschärfeverlusts für alle einzelnen Zernike-Koeffizienten
• Berechnen des VPDF für jeden einzelnen Zernike-Koeffizienten

$$\text{VPDF (Zx)} = (\text{mittlerer Sehschärfeverlust für Zx}) / (\text{mittlerer Sehschärfeverlust für Defokussierung});$$

(f) Berechnen der Restaberrationen;

(g) Berechnen der benötigten Vorderseitenkorrektur in Bezug auf Zernike-Koeffizienten;
(h) Umwandeln der Korrektur aus (g) unter Verwendung des die Sehleistung schädigenden Faktors, wobei der die Sehleistung schädigende Faktor wie in Schritt (e) vorangehend beschrieben berechnet wird;
(i) Beschaffen der relevanten Aberrationen höherer Ordnung für die Korrektur; und
(j) Beschaffen einer optimierten Gestalt für die Vorder- und die Rückseite der Linse.

3. Verfahren nach Anspruch 1 oder 2, wobei es sich bei der kundenspezifischen Linse um kontaktlinse, ein Inlay, ein Onlay oder eine intraokulare Linse handeln kann.

4. Verfahren nach Anspruch 3, wobei es sich bei der kundenspezifischen Linse um eine weiche oder eine starre Kontaktlinse handelt.

5. Verfahren nach eine der Ansprüche 1 bis 4, wobei die gesamten okularen Aberrationen höherer Ordnung unter Verwendung eines Wellenfrontsensors gemessen werden.

6. Verfahren nach Anspruch 1, wobei die Berechnung der Gestalt der Rückseite mit einer Annahme ausgeführt wird, dass die Hornhautaberrationen auf Null reduziert werden.

7. Verfahren nach Anspruch 1, wobei die Berechnung annimmt, dass die Rückseite der Linse neue Aberrationen erzeugt oder dass immer noch weitere Aberrationen von der Hornhautoberfläche vorliegen.

8. Verfahren nach Anspruch 2, wobei es sich bei der Berechnung der Restaberrationen in Schritt (g) um den Gesamtwert minus der Hornhautaberrationen handelt.

9. Verfahren nach Anspruch 2, wobei die Berechnung der Restaberrationen in Schritt (g) die Rückseitenaberrationen berücksichtigt.

10. Verfahren nach einem der Ansprüche 1 bis 9, wobei das Verfahren weiter kundenspezifisch angepasst ist, um die Pupillengröße der Versuchsperson zu berücksichtigen.

11. Verfahren nach einem der Ansprüche 1 bis 10, zusätzlich umfassend die folgenden Schritte: Ausstatten der Versuchsperson mit einer Versuchskontaktlinse, Messen der Kontaktlinsendezentrierung und dann entsprechend Ausgleichen.

12. Verfahren nach Anspruch 11, wobei die in Abwesenheit von Koaxialität zwischen der Kontaktlinse und der Pupille des Auges erzeugten Aberrationen berücksichtigt werden.

13. Verfahren nach Anspruch 1 oder 2, wobei der Schritt des Berechnens des Sehschärfeverlusts verglichen mit der Ausgangsleistung folgende Schritte umfasst:

(a) Zeigen einer Versuchsperson mindestens einer Sehprobentafel, wobei die Probentafel Bilder umfasst und zum Messen der visuellen Effekte von einzelnen oder Gruppen von Aberrationen höherer Ordnung geeignet ist, wobei die Bilder mit einzelnen oder Gruppen von Aberrationen höherer Ordnung von bekannten optischen Effekten verzerrt sind und die Lesbarkeit der Probentafel eine Angabe des visuellen Effekts der optischen Effekte bereitstellt;
(b) Notieren des visuellen Effekts der Verzerrungen durch Aufzeichnen der Lesbarkeit der Tafel; und
(c) Vergleichen der relativen Lesbarkeit derartiger verzerrter Tafeln durch eine Probenmenge von Versuchspersonen, die alle Tafeln lesen oder gelesen haben, um eine Angabe des visuellen Effekts der optischen Effekte bereitzustellen.

14. Verfahren nach Anspruch 13, wobei die Probentafel geeignet ist, um die Effekte von Aberrationen höherer Ordnung zu messen, wobei die Bilder mit Aberrationen höherer Ordnung von festen optischen Effekten deformiert sind.

15. Verfahren nach Anspruch 14, wobei die Bilder durch Defokussierung mit verschiedenen Effektivwerten verzerrt sind.

16. Verfahren nach Anspruch 14, wobei die Bilder durch optische Effekte mit verschiedenen Effektivwerten verzerrt sind.

17. Verfahren nach eine der Ansprüche 1 bis 16, wobei das Verfahren weiter den Schritt des Herstellens einer Linse umfasst, die der optimierten Linsengestalt entspricht.

**Revendications**

**1.** Un procédé de conception d'une lentille personnalisée possédant une surface arrière sphérique qui est adaptée pour l'effet visuel relatif de différents types d'aberrations comprenant les opérations suivantes :

(a) la mesure d'aberrations d'ordre supérieur oculaires totales pour une pupille donnée et pour des tailles de pupille spécifiques,
(b) le calcul de la correction de surface avant nécessaire en termes de coefficients de Zernike,
(c) la conversion de la correction au moyen du facteur préjudiciable à la performance visuelle (VPDF), où le facteur préjudiciable à la performance visuelle est calculé au moyen des opérations suivantes :

• le calcul d'une perte d'acuité visuelle comparée à une performance de base qui est l'acuité visuelle corrigée la meilleure pour des lettres à faible contraste et à contraste élevé, où l'acuité visuelle corrigée la meilleure est déterminée avec une réfraction sphéro-cylindrique correcte la meilleure, perte d'acuité visuelle = acuité visuelle corrigée la meilleure - performance visuelle mesurée
• le calcul de la perte d'acuité visuelle moyenne pour des mires à faible contraste et à contraste élevé
perte d'acuité visuelle moyenne = ((perte d'acuité visuelle pour contraste élevé) + (perte d'acuité visuelle pour faible contraste)) / 2
• le calcul de la d'acuité visuelle moyenne pour tous les coefficients de Zernike individuels
• le calcul du VPDF pour chaque coefficient de Zernike individuel VPDF (Zx) = (perte d'acuité visuelle moyenne due à Zx) / (perte d'acuité visuelle moyenne due à défocalisation),

(d) l'obtention des aberrations d'ordre supérieur pertinentes à des fins de correction, et
(e) l'obtention du mode de conception optimisé pour la surface avant de la lentille.

**2.** Un procédé dans lequel le VPDF est utilisé de façon à optimiser le mode de conception à la fois de la surface avant et de la surface arrière de la lentille comprenant les opérations suivantes :

(a) la mesure d'aberrations d'ordre supérieur oculaires totales pour une pupille donnée et pour des tailles de pupille spécifiques,
(b) la mesure d'aberrations oculaires générées par des irrégularités de la topographie de la cornée,
(c) le calcul du mode de conception de la surface arrière,
(d) le calcul de la correction de surface arrière nécessaire en termes de coefficients de Zernike,
(e) la conversion de la correction de (d) au moyen du facteur préjudiciable à la performance visuelle (VPDF), où le facteur préjudiciable à la performance visuelle est calculé au moyen des opérations suivantes :

• le calcul d'une perte d'acuité visuelle comparée à une performance de base qui est l'acuité visuelle corrigée la meilleure pour des lettres à faible contraste et à contraste élevé, où l'acuité visuelle corrigée la meilleure est déterminée avec une réfraction sphéro-cylindrique corrigée la meilleure, perte d'acuité visuelle = acuité visuelle corrigée la meilleure - performance visuelle mesurée
• le calcul de la perte d'acuité visuelle moyenne pour des mires à faible contraste et à contraste élevé

$$\text{perte d'acuité visuelle moyenne} = ((\text{perte d'acuité visuelle pour contraste élevé}) + (\text{perte d'acuité visuelle pour faible contraste})) / 2$$

• le calcul de la perte d'acuité visuelle moyenne pour tous les coefficients de Zernike individuels
• le calcul du VPDF pour chaque coefficient de Zernike individuel VPDF (Zx) = (perte d'acuité visuelle moyenne due à Zx) / perte d'acuité visuelle moyenne due à défocalisation),

(f) le calcul des aberrations résiduelles,
(g) le calcul de la correction de surface avant nécessaire en termes de coefficients de Zernike,
(h) la conversion de la correction de (g) au moyen du facteur préjudiciable à la performance visuelle, où le facteur préjudiciable à la performance visuelle est calculé comme décrit à l'opération (e) ci-dessus,
(i) l'obtention des aberrations d'ordre supérieur pertinentes à des fins de correction, et

(j) l'obtention d'un mode de conception optimisé pour la surface avant et la surface arrière de la lentille.

3. Le procédé selon la Revendication 1 ou 2 où la lentille personnalisée peut être une lentille de contact, un inlay, un onlay ou une lentille intraoculaire.

4. Le procédé selon la Revendication 3 où la lentille personnalisée est une lentille de contact souple ou rigide.

5. Le procédé selon l'une quelconque des Revendications 1 à 4 où les aberrations d'ordre supérieur oculaires totales sont mesurées au moyen d'un capteur de front d'onde.

6. Le procédé selon la Revendication 1 où le calcul du mode de conception de la surface arrière est effectué en faisant l'hypothèse que les aberrations de la cornée sont réduites à zéro.

7. Le procédé selon la Revendication 1 où le calcul fait l'hypothèse que la surface arrière de la lentille crée de nouvelles aberrations ou qu'il existe encore d'autres aberrations provenant de la surface de la cornée.

8. Le procédé selon la Revendication 2 où le calcul des aberrations résiduelles à l'opération (g) est le total moins les aberrations de la cornée.

9. Le procédé selon la Revendication 2 où le calcul des aberrations résiduelles à l'opération (g) prend en compte les aberrations de surface arrière.

10. Un procédé selon l'une quelconque des Revendications 1 à 9 où le procédé est en outre personnalisé de façon à prendre en compte la taille de pupille du sujet.

11. Un procédé selon l'une quelconque des Revendications 1 à 10 comprenant en outre les opérations d'équipement du sujet avec une lentille de contact d'essai, de mesure de l'excentrement de la lentille de contact et ensuite de compensation en conséquence.

12. Un procédé selon la Revendication 11 où les aberrations produites en l'absence de coaxialité entre la lentille de contact et la pupille de l'oeil sont prises en compte.

13. Le procédé selon la Revendication 1 ou 2, où l'opération de calcul d'une perte d'acuité visuelle comparée à une performance de base comprend les opérations suivantes :

(a) la présentation d'au moins une mire de test visuel à un sujet soumis à test, où la mire de test comprend des images et est adaptée à la mesure des effets visuels d'aberrations uniques ou de groupes d'aberrations d'ordre supérieur où les images sont déformées par des aberrations uniques ou des groupes d'aberrations d'ordre supérieur d'effets optiques connus et la lisibilité de la mire de test fournit une indication de l'effet visuel des effets optiques,
(b) la consignation de l'effet visuel des distorsions par l'enregistrement de la lisibilité de la mire, et
(c) la comparaison de la lisibilité relative de ces mires déformées par un panel de sujets soumis à test qui lisent ou ont lu toutes les mires de façon à fournir une indication de l'effet visuel des effets optiques.

14. Un procédé selon la Revendication 13 où la mire de test est adaptée à la mesure des effets d'aberrations d'ordre supérieur où les images sont déformées avec des aberrations d'ordre supérieur d'effets optiques fixes.

15. Un procédé selon la Revendication 14 où les images sont déformées par défocalisation de différentes valeurs RMS.

16. Un procédé selon la Revendication 14 où les images sont déformées par des effets optiques de différentes valeurs RMS.

17. Un procédé selon l'une quelconque des Revendications 1 à 16, où le procédé comprend en outre l'opération de production d'une lentille conforme au mode de conception de lentille optimisé.

## Fig.1.

## Fig.2.

## Fig.3.

## Fig.4.

## Fig.5.

## Fig.6.

**EP 1 597 623 B1**

REFERENCES CITED IN THE DESCRIPTION

**Patent documents cited in the description**

- US 6499843 B
- US 6086204 A
- US 6338559 B
- US 6305802 B
- US 6449843 B